(19) Europäisches Patentamt
European Patent Office
Office européen des brevets

(11) Publication number: 0 171 942
A1

(12) **EUROPEAN PATENT APPLICATION**

(21) Application number: 85305166.2

(22) Date of filing: 19.07.85

(51) Int. Cl.[4]: A 61 K 31/70
//(A61K31/70, 31:505)

(30) Priority: 19.07.84 JP 151063/84

(43) Date of publication of application:
19.02.86 Bulletin 86/8

(84) Designated Contracting States:
AT BE CH DE FR GB IT LI LU NL SE

(71) Applicant: Takeda Chemical Industries, Ltd.
27, Doshomachi 2-chome Higashi-ku
Osaka-shi Osaka, 541(JP)

(72) Inventor: Ootsu, Koichiro
9-26, Todaiji 3-chome Shimamoto-cho
Mishima-gun Osaka 618(JP)

(72) Inventor: Okutani, Tetsuya
3-5, Matsugaoka 4-chome
Takatsuki Osaka 569(JP)

(74) Representative: Lewin, John Harvey et al,
Elkington and Fife High Holborn House 52/54 High Holborn
London WC1V 6SH(GB)

(54) **Antitumor composition.**

(57) An antitumor composition was prepared by admixing a 5-fluorohexahydropyrimidine of the formula (I)

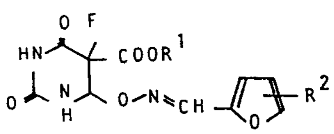

(1)

wherein $R^1$ is a lower alkyl group and $R^2$ is a hydrogen atom or a lower alkyl group, and a pyrimidine nucleic acid base and/or the riboside thereof. Thus, the antitumor activity of the 5-fluorohexahydropyrimidine of the formula (I) is remarkably increased by the joint use of a pyrimidine nucleic acid base and/or the riboside thereof, which has little antitumor activity.

EP 0 171 942 A1

Antitumor Composition

This invention relates to an antitumor composition containing a 5-fluorohexahydropyrimidine and a pyrimidine nucleic acid base and/or the riboside thereof.

5-Fluorouracil is an antitumor agent synthesized by Duschinsky [J.Amer.Chem.Soc. 79,4559(1957)]. Basic and clinical investigations of the same as an antitumor antimetabolite by Heidelberger et al.[Nature 179,663(1957)] and Ansfield et al.[Canser Chemotherapy Reports 32,101(1963)] have revealed that it has a broad antitumor spectrum. Since then, 5-fluorouracil has become one of the antitumor agents in widest use in the treatment of various adenocarcinomas, inclusive of digestive organ cancer and breast cancer, and so forth. In Japan, the so-called masked type 5-fluorouracil compounds, such

as 1-(2-tetrahydrofurfuryl)-5-fluorouracil and 5-fluoro-1-hexylcarbamoyluracil, have been studied intensively in the attempt to reduce the toxic effects encountered when 5-fluorouracil is directly administered and, for some of them, therapeutic regimes for oral administration have already been established clinically.

On the other hand, Japanese Published unexamined patent Laid-open Nos. 55-7,231, 55-64,518, 56-46,813, 56-46,814 and 56-99,414 disclose that the antitumor effect of 5-fluorouracils can be potentiated by uracils.

It is an object of the invention to provide a useful antitumor composition with which the antitumor activity of 5-fluorohexahydropyrimidines (Japanese Published unexamined patent Laid-open No. 59-13,780) having the general formula (I)

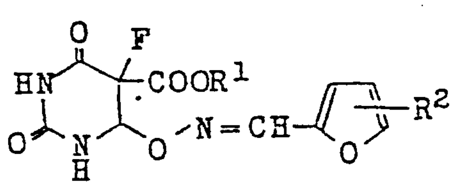

( I )

wherein $R^1$ is a lower alkyl group and $R^2$ is a hydrogen atom or a lower alkyl group, can be further potentiated.

Under these circumstances, the present inventors conducted intensive investigations and, as a result, found that the antitumor activity of the compounds of general formula (I) can be significantly potentiated by concomitant administration of a pyrimidine nucleic acid base

or the riboside thereof. This finding and further investigations have now resulted in completion of the present invention.

Thus, the invention consists in an antitumor composition which contains a fluorohexahydropyrimidine of general formula (I) and a pyrimidine nucleic acid base and/or the riboside thereof.

The 5-fluorohexahydropyrimidine to be used in accordance with the invention is a compound represented by the above general formula (I), and a wide variety of compounds of this formula can be used. Referrring to general formula (I), the lower alkyl represented by $R^1$ and that represented by $R^2$ may be the same or different and each is, for instance, an alkyl containing 1 to 6 carbon atoms, for example,methyl, ethyl, propyl, isopropyl, butyl, isobutyl, secondary butyl, tertiary butyl, pentyl, isopentyl or hexyl. With respect to the furfurylideneaminooxy group in position 4, and also with respect to the fluorine atom or aminoalkoxycarbonyl group in position 5 and the furfurylideneaminooxy group in position 4, there can occur cis- and trans-stereoisomers, and such respective isomers and mixtures thereof fall within the scope of general formula (I), hence of the present invention.

Among the compounds of general formula (I), there are preferably used the following compounds, for instance:

- o Ethyl 5-fluoro-4-[(Z)-furfurylideneaminooxy]-2,6-dioxohexahydropyrimidine-5-carboxylate (compound 1);
- o Ethyl 5-fluoro-4-[(E)-furfurylideneaminooxy]-2,6-dioxohexahydropyrimidine-5-carboxylate (compound 2);
- o Isobutyl 5-fluoro-4-[(Z)-furfurylideneaminooxy]-2,6-dioxohexahydropyrimidine-5-carboxylate (compound 3);
- o Isobutyl 5-fluoro-4-[(E)-furfurylideneaminooxy]-2,6-dioxohexahydropyrimidine-5-carboxylate (compound 4);
- o Ethyl 5-fluoro-4-[(Z)-(5-methyl)furfurylideneaminooxy]-2,6-dioxohexahydropyrimidine-5-carboxylate (compound 5);
- o Isopropyl 5-fluoro-4-[(E)-furfurylideneaminooxy]-2,6-dioxohexahydropyrimidine-5-carboxylate (compound 6); and
- o Isopropyl 5-fluoro-4-[(Z)-furfurylideneaminooxy]-2,6-dioxohexahydropyrimidine-5-carboxylate (compound 7).

The compounds of general formula (I) are all known compounds, and a method of producing the same is described, for example, in Japanese Published unexamined patent Laid-open No. 59-13,780. In the antitumor composition according to the invention, the 5-fluorohexahydropyrimidines of general formula (I) may be incorporated either alone or in combination of two or more.

The term "pyrimidine nucleic acid base" as used herein means a pyrimidine base contained in nucleic acids and includes uracil, cytosine and thymine, and the riboside thereof includes uridine, cytidine, thymidine and 2'-deoxy

derivatives of these. Of these compounds, one or more are selected for use.

The proportion of the 5-fluorohexahydropyrimidine to the pyrimidine nucleic acid base and/or riboside thereof in the antitumor composition according to the invention may vary depending on the kind of each compound contained therein, the dosage form, and other factors. Generally, however, the pyrimidine nucleic acid base and/or riboside thereof is used in an amount of not less than 0.5 mole, preferably not less than 1 mole, more preferably not less than 2 moles, per mole of the compound of general formula (I). Although the antitumor effect increases with the increase in the proportion of the pyrimidine nucleic acid base and/or riboside thereof, said base and/or riboside is generally used in a proportion of not more than about 20 moles, preferably not more than about 15 moles, per mole of the compound of general formula (I).

Even when the 5-fluorohexahydropyrimidine and the pyrimidine nucleic acid base and/or riboside thereof are administered separately, the antitumor activity can be increased. It is preferable, however, to administer the both simultaneously in the form of a mixture prepared in advance. The unit dosage form of the antitumor composition according to the invention may be selected from among various forms depending on the therapeutic purpose. For example, there may be mentioned dosage forms for oral administration, such as powders, tablets, capsules, granular preparations and liquid preparations, and dosage forms for parenteral administration, such as suppositories and injections. In making up the composition into such unit dosage forms, any appropriate carrier known in the art can be used and the forming can be carried out by an appropriate means commonly employed in the art.

The amount of the 5-fluorohexahydropyrimidine to be incorporated in such various unit dosage forms varies depending on the kind of said compound itself as well as on the kind of the pyrimidine nucleic acid base and/or riboside thereof to be used combinedly and therefore cannot be specified definitely. Generally, however, said compound is used in an amount, per dosage unit, of about 5-600 mg for oral preparations, about 5-300 mg for injections, or about 10-800 mg for suppositories, the pyrimidine nucleic acid base and/or riboside thereof being used, for example, in the proportion mentioned above based on said amount of 5-fluorohexahydropyrimidine. The daily dose, too, depends on the kind of said 5-fluorohexahydropyrimidine and the proportion of the pyrimidine nucleic acid base and/or riboside thereof, hence cannot be specifically given. Generally, however, it is desirable, in the estimation from the results of fundamental pharmacological efficacy tests, that the daily clinical dose be in the range of about 0.4-12 mg/kg body weight for oral preparations, about 0.2-6 mg/kg body weight for injections, or about 0.8-16 mg/kg body weight for suppositories.

A characteristic feature of the present invention lies in that the antitumor activity of the 5-fluorohexahydropyrimidine of general formula (I) is remarkably increased by the joint use of a pyrimidine nucleic acid base and/or the riboside thereof, which each itself has little antitumor activity.

Thus, the antitumor composition according to the invention can be safely administered either orally or parenterally and produce a very great antitumor effect as compared with the case of single administration of the 5-fluorohexahydropyrimidine of general formula (I) and therefore can inhibit the growth of tumor cells and produce a significant life span-prolonging effect in cancer-bearing warm-blooded animals (e.g. rat, mouse, human). Thus, for example, the composition according to the invention is useful as an antitumor agent for varieties of solid cancer (e.g. digestive organ cancer, lung cancer, breast cancer, urinary organ cancer).

For further illustration of the present invention, several formulation examples of the antitumor composition according to the invention are given below together with two antitumor effect test examples.

Formulation Example 1

| | |
|---|---|
| Compound 1 | 100 mg |
| Uracil | 100 mg |
| Lactose | 70 mg |
| Crystalline cellulose | 27 mg |
| Magnesium stearate | 3 mg |
| Per capsule | 300 mg |

In accordance with the above formulation, dosage form capsules are prepared by a conventional method.

Formulation Example 2

| | |
|---|---|
| Compound 7 | 100 mg |
| Uracil | 150 mg |
| Crystalline cellulose | 35 mg |
| Magnesium stearate | 2 mg |
| Talc | 3 mg |
| Hydroxypropylcellulose | 10 mg |
| Per tablet | 300 mg |

In accordance with the above formulation, tablets are prepared by a conventional method.

Formulation Example 3

| | |
|---|---|
| Compound 5 | 200 mg |
| Thymine | 400 mg |

| | |
|---|---|
| Lactose | 330 mg |
| Corn starch | 40 mg |
| Hydroxypropylmethylcellulose | 30 mg |
| Per pack | 1,000 mg |

In accordance with the above formulation, a granular dosage form is prepared by a conventional method.

Formulation Example 4

| | |
|---|---|
| Compound 3 | 600 mg |
| Uracil | 400 mg |
| WITEPSOL W-35 (Dynamit Nobel, West Germany) | 1,000 mg |
| Per suppository | 2,000 mg |

In accordance with the above formulation, suppositories are prepared by a conventional method.

Formulation Example 5

| | |
|---|---|
| Compound 4 | 150 mg |
| Uracil | 450 mg |
| Lactose | 330 mg |
| Corn starch | 40 mg |
| Hydroxypropylmethylcellulose | 30 mg |
| Per pack | 1,000 mg |

In accordance with the above formulation, a granular dosage form is prepared by a conventional method.

Formulation Example 6

| | |
|---|---|
| Compound 2 | 50 mg |
| Uridine | 200 mg |
| Lactose | 70 mg |

| | |
|---|---|
| Crystalline cellulose | 27 mg |
| Magnesium stearate | 3 mg |
| Per capsule | 350 mg |

In accordance with the above formulation, capsules are prepared by a conventional method.

Formulation Example 7

| | |
|---|---|
| Compound 6 | 200 mg |
| Thymidine | 400 mg |
| Lactose | 330 mg |
| Corn starch | 40 mg |
| Hydroxypropylmethylcellulose | 30 mg |
| Per pack | 1,000 mg |

In accordance with the above formulation, a granular dosage form is prepared by a conventional method.

Formulation Example 8

| | |
|---|---|
| Compound 3 | 200 mg |
| Uridine | 600 mg |
| Lactose | 130 mg |
| Corn starch | 40 mg |
| Hydroxypropylmethylcellulose | 30 mg |
| Per pack | 1,000 mg |

In accordance with the above formulation, a granular dosage form is prepared by a conventional method.

Test Example 1

The antitumor composition according to the invention was tested for antitumor effect using Lewis lung carcinoma. Thus, each female $F_1$ mouse [C57BL/6 x DBA/2] weighing 18 ± 1 g was implanted with a tumor fragment about 2 $mm^3$ in volume subcutaneously into the axillary site using a trocar. Starting from 24 hours after tumor implantation, the above-mentioned compound 4 and uracil were orally administered combinedly in the proportion given below in Table 1 once daily for 7 days, and the antitumor effect was compared with that attained when each of them was administered singly. The sample agent was suspended in a 0.5% solution of gum arabic in physiological saline just before daily administration such that the dose amounted to 0.2 ml/20 g body weight. Ten days after tumor implantation, the tumor was excised and weighed, and the average tumor weight ratio (T/C %) between the dosed group (T, group of 5 animals) and the control group (C, group of 10 animals) was calculated. The daily dose was expressed in terms of number of moles per kilogram of body weight (mM/kg).

Table 1

| Dose of compound 4 (mM/kg) | Dose of uracil (mM/kg) | Moles of uracil per mole of compound 4 | Tumor weight (mg) | T/C (%) |
|---|---|---|---|---|
| 0 | 0 | | 255 | |
| 0.1 | 0 | | 138 | 54 |
| 0.2 | 0 | | 107 | 42 |
| 0.4 | 0 | | 80 | 31 |
| 0.8 | 0 | | 49 | 19 |
| 0 | 0.4 | | 319 | 125 |
| 0 | 0.8 | | 572 | 224 |
| 0 | 1.6 | | 502 | 197 |
| 0 | 3.2 | | 432 | 169 |
| 0.1 | 0.4 | 4 | 102 | 40 |
| 0.2 | 0.8 | 4 | 52 | 20 |
| 0.4 | 1.6 | 4 | 29 | 11 |
| 0.8 | 3.2 | 4 | 21 | 8 |

Test Example 2

The antitumor composition according to the invention was further tested for antitumor effect using colon carcinoma 26. Thus, each female $F_1$ mouse [BALB/c x DBA/2] weighing 21 ± 1 g was subcutaneously implanted with a tumor fragment (about 2 $mm^3$) into the axillary site using a trocar. Starting from 24 hours after tumor implantation, the above-mentioned compound 4 and uracil were orally administered combinedly on the proportion given below in Table 2 once a day for 7 days, and the antitumor effect was compared with that attained by single administration of either of them. The test agent was suspended in a 0.5% solution of gum arabic in physiological saline daily just before administration such that the dose amounted to 0.2 ml/20 g body weight. Ten days after tumor implantation, the tumor was excised and weighed, and the average tumor weight ratio (T/C %) between the dosed group (T, group of 5 animals) and the control group (C, group of 20 animals) was calculated. The daily dose was expressed in terms of number of moles per kilogram of body weight (mM/kg).

Table 2

| Dose of compound 4 (mM/kg) | Dose of uracil (mM/kg) | Moles of uracil per mole of compound 4 | Tumor weight (mg) | T/C (%) |
|---|---|---|---|---|
| 0 | 0 | | 198 | |
| 0.1 | 0 | | 190 | 96 |
| 0.2 | 0 | | 57 | 29 |
| 0.4 | 0 | | 2 | 1 |
| 0.8 | 0 | | 0 | 0 |
| 0 | 0.4 | | 223 | 113 |
| 0 | 0.8 | | 173 | 87 |
| 0 | 1.6 | | 170 | 86 |
| 0 | 3.2 | | 194 | 98 |
| 0.1 | 0.4 | 4 | 62 | 31 |
| 0.2 | 0.8 | 4 | 2 | 1 |
| 0.4 | 1.6 | 4 | 0 | 0 |

From the results shown in Table 1 and Table 2, it was noted that the combination of compound 4 and uracil causes a marked increase in antitumor effect. It was also revealed that the combined use of uracil enables compound 4 to produce an antitumor effect even in low doses in which

said compound, when used alone, cannot produce any antitumor effect.

As the above test results indicate, the antitumor composition according to the invention is a very excellent antitumor agent much improved in antitumor activity as compared with the 5-fluorohexahydropyrimidine compound of general formula (I) used alone.

What we claim is:

1. An antitumor composition which contains a 5-fluorohexahydropyrimidine of the formula

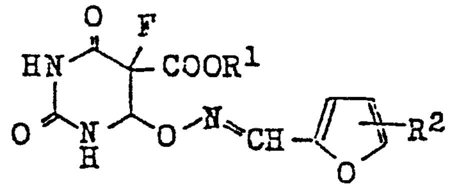

wherein $R^1$ is a lower alkyl group and $R^2$ is a hydrogen atom or a lower alkyl group, and a pyrimidine nucleic acid base and/or the riboside thereof.

2. The coposition according to Claim 1, wherein $R^1$ and $R^2$ are alkyls of 1 to 6 carbon atoms.

3. The composition according to Claim 1, wherein $R^2$ is hydrogen.

4. The composition according to Claim 1, wherein $R^1$ is ethyl, isobutyl or isopropyl and $R^2$ is hydrogen or methyl.

5. The composition according to Claim 1, wherein the pyridine nucleic acid base is uracil, cytosine or thymine, and the riboside thereof is uridine, cytidine or thymidine.

6. The composition according to Claim 1, wherein the proportion of the pyrimidine nucleic acid base and/or riboside thereof is about 1 to 15 moles per mole of 5-fluorohexahydropyrimidine.

7. The composition according to Claim 1, wherein the 5-fluorohexahydropyrimidine is isobutyl 5-fluoro-4-[(E)-furfurylideneaminooxy]-2,6-dioxohexahydropyrimidine-5-carboxylate and the pyrimidine nucleic acid base is uracil.

8. The composition according to Claim 1, wherein the 5-fluorohexahydropyrimidine is isobutyl 5-fluoro-4-[(E)-furfurylideneaminooxy]-2,6-dioxohexahydropyrimidine-5-carboxylate and the pyrimidine nucleic acid base is cytosine.

9. The composition according to Claim 1, wherein the 5-fluorohexahydropyrimidine is isobutyl 5-fluoro-4-[(E)-furfurylideneaminooxy]-2,6-dioxohexahydropyrimidine-5-carboxylate and the pyrimidine nucleic acid base is thymine.

10. The composition according to Claim 1, wherein the composition is in the form of powder, granule, tablet, capsule or suppository.

Claims for contracting state: AT (Austria)

What we claim is:

1. A method for preparing an antitumor composition which comprises incorporating therein a 5-fluorohexahydro-pyrimidine of the formula

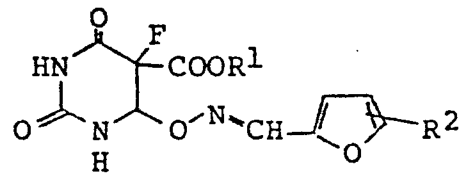

wherein $R^1$ is a lower alkyl group and $R^2$ is a hydrogen atom or a lower alkyl group, and a pyrimidine nucleic acid base and/or the riboside thereof.

2. A method according to claim 1, wherein $R^1$ is an alkyl of 1 to 6 carbon atoms.

3. A method according to claim 1, wherein $R^2$ is hydrogen.

4. A method according to claim 2, wherein the alkyl is isobutyl.

5. A method according to claim 1, wherein the pyridine nucleic acid base is uracil, cytosine or thymine.

6. A method according to claim 1, wherein the riboside is uridine, cytidine or thymidine.

7. A method according to claim 1, wherein the proportion of the pyrimidine nucleic acid base and/or riboside thereof is about 1 to 15 moles per mole of the 5-fluorohexahydropyrimidine.

8. A method for preparing a composition for increasing antitumor activity which comprises incorporating therein a 5-fluoro-hexahydropyrimidine of the formula

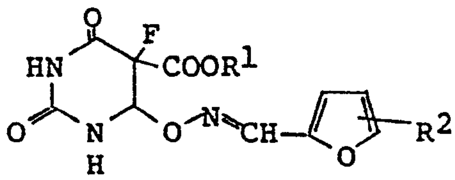

wherein $R^1$ is a lower alkyl group and $R^2$ is a hydrogen atom or a lower alkyl group, and a pyrimidine nucleic acid base and/or the riboside thereof.

European Patent Office

# EUROPEAN SEARCH REPORT

0171942

Application number

EP 85 30 5166

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | EP-A-0 099 091 (TAKEDA CHEMICAL INDUSTRIES LTD.) * Pages 17-19; claims 1-16 * ----- | 1-10 | A 61 K 31/70 // (A 61 K 31/70 A 61 K 31:505) |
| | | | TECHNICAL FIELDS SEARCHED (Int. Cl.4) |
| | | | A 61 K |

The present search report has been drawn up for all claims

| Place of search | Date of completion of the search | Examiner |
|---|---|---|
| THE HAGUE | 22-10-1985 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82